# EUROPEAN PATENT SPECIFICATION

(11) **EP 1 663 092 B1**
(45) Date of publication and mention of the grant of the patent: **23.11.2011**
(21) Application number: 04775407.2
(22) Date of filing: 10.09.2004
(51) Int. Cl.: A61F 13/15, A61F 13/62, A61F 13/56, A61F 13/58, A41B 13/00

(54) **ABSORBENT ARTICLE COMPRISING A FASTENING ARRANGEMENT**
SAUGFÄHIGER ARTIKEL MIT EINER BEFESTIGUNGSANORDNUNG
ARTICLE ABSORBANT COMPRENANT UN AGENCEMENT DE FIXATION

(30) Priority: 12.09.2003 SE 0302433
(43) Date of publication of application: 07.06.2006
(73) Proprietor: SCA Hygiene Products AB, 405 03 Göteborg (SE)
(72) Inventor: HERMANSSON, Kent, S-426 54 Västra Frölunda (SE); ESPING ÖSTLIN, Hanna, S-435 43 PIXBO (SE); CLASSON, Maria, S-436 45 Askim (SE)
(74) Representative: Romare, Laila Anette
(86) International application number: PCT/SE2004/001302
(87) International publication number: WO 2005/025472

(56) References cited:
- WO-A1-99/23904
- WO-A2-02/11657
- US-A- 5 289 619
- US-A- 5 318 555
- US-A1- 2002 078 536

## Description

### TECHNICAL FIELD

The present invention relates to an absorbent article such as a diaper, an incontinence pad or a sanitary towel, comprising an absorbent body, a covering comprising a liquid-permeable covering layer and a backing layer, and also a fastening arrangement for fastening the article on a wearer. In this connection, the fastening arrangement comprises a first and a second fastening device. Each fastening device comprises a female part and a male part, the female part having a first projecting tongue connected to the covering, and a second projecting tongue connected to the covering, a surface on the first projecting tongue being oriented towards a surface on the second projecting tongue, and a first fixing element being arranged on that surface of the first projecting tongue which is oriented towards the second projecting tongue, and a second fixing element being arranged on that surface of the second projecting tongue which is oriented towards the first projecting tongue. The male part is connected to the covering and has a first surface comprising a third fixing element, and a second surface comprising a fourth fixing element. The male part and the female part are interconnectable, the first fixing element interacting with the third fixing element and the second fixing element interacting with the fourth fixing element.

The invention also relates to a method for fitting an absorbent article comprising a fastening arrangement according to the invention on a wearer.

### BACKGROUND ART

Fastening arrangements for absorbent articles often consist of attachment flaps or attachment tabs which are positioned in the rear side portions of the article and of a receiving part arranged on the front portion of the article. Arranged on the attachment flap is an attachment means such as, for example, tape or a male part or female part of a hook and loop tape, and, when the absorbent article is attached around a wearer, the article is stretched around the waist of the wearer and the attachment means of the attachment flaps are attached to the receiving part on the front side of the article. Examples of attachment tabs are given in GB 2,311,096 B, WO 95/16425 A2, WO 96/19960 A1, US 5,738,930 A, EP 0,235,014 B1 and EP 0,321,234 A1.

A more complex fastening arrangement is described in the document WO 02/11657 A2. The fastening arrangement described in this document comprises two receiving elements arranged in one of the waist regions of the article, the receiving elements having a first and a second projecting tongue anchored to the covering of the article. Fixing elements are arranged on those surfaces of the projecting tongues which are arranged facing one another.

Arranged in the opposite waist region of the article are two attachment tabs, the tabs being adapted so as to be interconnected with the receiving elements. The attachment tabs have fixing elements on both their opposite surfaces.

When the article is fitted on a wearer, the attachment tabs are fixed between the projecting tongues of the receiving elements, the fixing elements on each projecting tongue being interconnected with the fixing elements on that surface of the respective attachment tab which is arranged facing the tongue.

Both the projecting tongues of the receiving elements and the attachment tabs can have fixing elements of various kinds such as hooks and loops and/or adhesive arrangements.

Each projecting tongue and each attachment tab can comprise a number of fixing elements. The use of a number of fixing elements is especially common when the receiving elements and the attachment tabs are wide, adapted for fixing a wider waist region. In this connection, each receiving element and attachment tab usually has a first fixing element adjacent to one of its longitudinal edges and a second fixing element adjacent to its other longitudinal edge. A region between the fixing elements often has no fixing element, but a third fixing element may be arranged between the first two fixing elements. This type of wide fastening arrangement is especially common for what are known as reclosable absorbent articles of pant type.

One problem with the complex fastening arrangements described above is that they are difficult to fit so that the result is aesthetically attractive. It is usual, especially when the attachment system has a great width, for the projecting tongues of the receiving elements or the attachment tabs to comprise more than one fixing element arranged one beside another in the width direction of the tongue or attachment tab. In such arrangements, it is a problem to join the fixing elements of the receiving elements together with the fixing elements of the attachment tabs in such a way that smooth surfaces are obtained when the whole attachment system is fixed. Both hands normally have to be used for interconnecting such systems.

When an absorbent article is to be fitted on an adult incontinent wearer who is confined to bed, a common procedure is first to position the rear part of the absorbent article under the buttocks of the wearer, to fold the front part of the article over the abdomen of the wearer, and then to begin the interconnection of the front and rear waist regions of the article. In this regard, the waist regions on that side of the article/the patient where the carer is located are normally interconnected first, after which the patient is rolled through a quarter to half a turn in the bed so that the second waist region becomes accessible and can be interconnected. Going around the bed in which the patient is lying is considered too time-consuming by most carers.

The fitting procedure is in principle the same when fitting takes place on a standing wearer.

Interconnection of the waist regions of the absorbent article according to the procedure described above means that interconnection of the waist region on the first and the second side of the article is carried out on mirror-inverted components as far as the fitter is concerned, the operations performed with the right hand when the first side of the article was interconnected expediently having to be performed with the left hand when the second side of the article is interconnected.

For conventional, uncomplicated fastening arrangements, this does not involve any problems, because the necessary operations are few and simple and can be performed with one hand.

In the case of more complex fastening arrangements as above where a number of fixing elements have to be interconnected with one another in a certain order, where both hands have to be used for fitting, and where various parts of the fixing tabs and the receiving elements, for example, have to be stretched out before interconnection of the next fixing elements in the fixing sequence, working in a mirror-inverted way for the two sides of the article involves difficulties.

### DISCLOSURE OF INVENTION

With the present invention, however, an absorbent article comprising a fastening arrangement of the kind referred to in the introduction has been produced, with a first and a second fastening device, each comprising a two-part female part and a two-part male part, which article essential eliminates the problems which were associated with previously known such products.

In this connection, an absorbent article made according to the invention in accordance with a first embodiment is characterized mainly in that the respective female parts of the fastening devices are arranged in diagonally opposite corners of the article, and in that the respective male parts of the fastening devices are arranged in the other two diagonally opposite corners of the article when the article is in a folded-out state.

According to a second embodiment, the fastening arrangement of the absorbent article comprises a fifth fixing element arranged on that surface of the first projecting tongue of the female part which is oriented towards the second projecting tongue. The fastening arrangement of the article also comprises a sixth fixing element arranged on the first surface of the male part, the fifth and sixth fixing elements interacting with one another.

According to one embodiment, the fastening devices can comprise mechanical fixing elements of various kinds.

A particularly preferred form of mechanical fixing element consists of hook and loop elements, the male parts and female parts of the hook and loop elements being arranged in a suitable manner on the various parts of the fastening devices.

According to another embodiment, the fastening devices of the fastening arrangement can comprise adhesive fixing elements.

Combinations of mechanical and adhesive fixing elements are also a conceivable embodiment.

In an alternative embodiment, the male parts of the fastening devices can comprise the liquid-permeable covering layer of the incontinence pad or the backing layer, or both layers. In combination therewith, or alternatively, the female parts of the fastening devices can also comprise the liquid-permeable covering layer of the incontinence pad or the backing layer, or both layers.

According to one embodiment, the absorbent article consists of an openable pant product.

The present invention also relates to a method for fitting an absorbent article comprising a fastening arrangement of the kind referred to in the introduction, which method essentially eliminates the problems which were associated with previously known such methods.

In this connection, a characteristic method according to the invention is characterized mainly in that interconnection of the two fastening devices of the fastening arrangement is carried out from the same position in relation to the article and the wearer of the article, the sequence of operations for interconnection of the fastening device selected first and the fastening device selected subsequently being identical, the same hand being used for the same operations in the sequence for both the fastening device selected first and the fastening device selected subsequently.

According to a preferred method, one fastening device of the fastening arrangement is interconnected first. In this regard, as a first step, the first fixing element is interconnected with the third fixing element, after which the second fixing element of the first fastening device is interconnected with the fourth fixing element.

The fixing elements of the second fastening device are then interconnected in the same order as the order for the fixing elements of the first fastening device.

Another preferred method comprises as a first step interconnecting one fastening device, its first fixing element being interconnected with the third fixing element, followed by interconnection of the fifth and sixth fixing elements. The interconnection of the first fastening device is then completed by interconnection of its second and fourth fixing elements. The fitting is then finished by the second fastening device being interconnected in the same order as the order for the fixing elements of the first fastening device.

An alternative method comprises as a first step interconnecting one fastening device, its fifth fixing element being interconnected with the sixth fixing element, followed by interconnection of the first and third fixing elements. The interconnection of the first fastening device is then completed by interconnection of its second and fourth fixing elements. The fitting is then finished by the second fastening device being interconnected in the same order as the order for the fixing elements of the first fastening device.

### BRIEF DESCRIPTION OF FIGURES

- Figure 1: shows an absorbent article comprising a fastening arrangement according to the invention in the folded-out state from the side which is intended to face the wearer during use.
- Figure 2: shows an absorbent article comprising a fastening arrangement according to the invention in a configuration immediately before interconnection of the two fastening devices.
- Figure 3: shows an enlarged illustration of a first embodiment of the two fastening devices of the absorbent article before their interconnection.
- Figure 4: shows an enlarged illustration of a second embodiment of the two fastening devices of the absorbent article before their interconnection.

### DESCRIPTION OF ILLUSTRATIVE EMBODIMENTS

The invention relates to an absorbent article such as a baby diaper, an incontinence pad or a sanitary towel comprising a fastening arrangement.

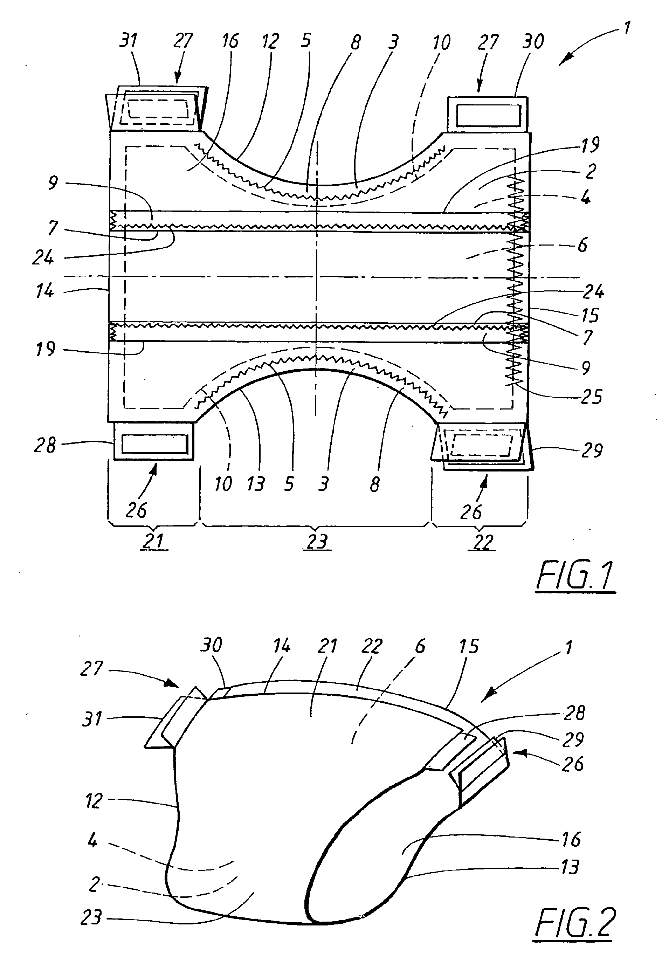

The embodiment shown in Figure 1 and Figure 2 relates to an incontinence pad 1 for adult wearers who suffer from heavier forms of incontinence.

The incontinence pad 1 is essentially hourglass-shaped and has longitudinal edges 12, 13, a front transverse edge 14 and a rear transverse edge 15, and also a front and a rear end portion 21, 22 and a narrower crotch portion 23 located between the end portions 21, 22. During use, the crotch portion 23 is intended to be located in the narrowest region between the thighs of the wearer.

During use of the incontinence pad 1, the front part of the crotch portion 23 and the front end portion 21 function mainly as a receiving area for urine, while the rear part of the crotch portion 23 and the rear end portion 22 function mainly as a receiving area for faecal matter.

The incontinence pad 1 comprises a covering layer 16 comprising a liquid-permeable covering layer 2, arranged over that surface on the incontinence pad 1 which is intended to face the wearer during use, and a backing layer 4 arranged over that surface on the article which is intended to face away from the wearer during use, an absorbent body 6 enclosed between the liquid-permeable covering layer 2 and the backing layer 4, and side flaps 3 arranged outside the absorbent body 6.

The liquid-permeable covering layer 2 extends outside the absorbent body 6 along the entire periphery of the absorbent body 6. The liquid-permeable covering layer 2 can consist of any material suitable for the purpose. Examples of common liquid-permeable covering materials are non-woven textile materials, perforated plastic films, nets made of plastic or textile, and liquid-permeable foam layers. Liquid-permeable covering materials which consist of continuous thin fibres which extend mainly in the longitudinal or transverse direction of the article also exist. Laminates consisting of two or more of the possible covering materials mentioned above are also common, as are coverings consisting of different materials within different parts of the surface.

Absorbent articles comprising absorbent bodies 6 which have particularly high strength and wear-resistance can even function without any extra liquid-permeable covering layer being required on that side of the article which faces the wearer during use.

The backing layer 4 also extends outside the absorbent body 6 along the entire periphery of the absorbent body 6. Normal backing layers on absorbent articles should be liquid-impermeable, but other types of backing layer also exist. The backing layer 4 can consist of a number of different materials. It is most common for the backing layer 4 to consist of a thin liquid-impermeable plastic film, but it is also possible to use other types of liquid-impermeable material, such as non-woven materials which have been made liquid-impermeable by, for example, plastic coating, liquid-impermeable foam layers, liquid-impermeable adhesive or the like. The backing layer 4 can also consist of a vapour-permeable material. Laminates consisting of at least one liquid-impermeable material also exist. These laminates usually consist of a liquid-impermeable material functioning as a liquid barrier and a more textile-like material arranged on that side of the article which is oriented away from the wearer during use, the outside of the article then being more clothing-like.

The liquid-permeable covering layer 2 and the backing layer 4 are interconnected outside the absorbent body 6 along the entire periphery of the absorbent body 6.

The liquid-permeable covering layer 2 and the backing layer 4 can be interconnected in a number of different ways. Examples of connecting methods are gluing, thermal welding, ultrasonic welding or the like.

Elastic means 5 are arranged outside the absorbent body 6 in those parts of the side flaps 3 of the incontinence pad 1 which essentially run in the longitudinal direction of the incontinence pad 1. The elastic means 5 function as leg elastic and have the task of preventing liquid and motions leaking out past the side edges 12, 13 running in the longitudinal direction and in this way, together with surrounding layers, form outer liquid barriers 8. The elastic means 5 consist of one or more elastic threads which have, in the stretched state, been applied between the liquid-permeable covering layer 2 and the backing layer 4, at least in the crotch portion 23 of the incontinence pad 1. The elastic means 5 are connected to the backing layer 4 and the covering layer 2 by gluing, ultrasonic welding or.the like.

In alternative embodiments, the elastic means can be arranged on the side of the side flaps 3 which is intended to face the wearer during use, or on the opposite side of the side flaps, and are then of course connected to only the covering layer 2 or, respectively, the backing layer 4.

In alternative embodiments, the elastic means can consist of elastic band materials made of, for example, foam material.

The hourglass-shaped absorbent body 6 can be constructed from one or more layer(s) of cellulose fluff pulp. In this connection, the cellulose fluff pulp can be mixed with fibres or particles of a highly absorbent polymer material of the kind which, during absorption, chemically binds great quantities of liquid while forming a liquid-containing gel. The absorbent body 6 can also comprise highly absorbent polymer material arranged in a layer inside the absorbent body or adjacent to the surface or surfaces of the absorbent body. The absorbent body 6 can also include further components for improving the properties of the absorbent body 6. Examples of such components are bonding fibres, various types of liquid-spreading layer or fibre, shape-stabilizing components, strengthening fibres or the like. The absorbent body 6 can of course also consist of other types of absorbent material, such as absorbent non-woven materials, absorbent foams, textile materials, peat or mixtures of different kinds of absorbent material.

Special layers for rapidly receiving great quantities of liquid and temporarily retaining this liquid in order then to transfer the temporarily stored liquid to other parts of the absorbent body 6 can also be included in incontinence pads of the type indicated. Such receiving layers are then normally arranged between the liquid-permeable covering layer 2 and absorbent body 6 of the incontinence pad 1. No receiving layer is illustrated in any of the figures.

In order further to prevent liquid or faecal matter leaking out over the side edges 12, 13 of the incontinence pad 1, the incontinence pad 1 is provided with inner side leakage barriers 9 on the side which is intended to face the wearer during use. The inner side leakage barriers 9 are arranged adjacent to the longitudinal edges 10 of the absorbent body 6 and extend essentially in the longitudinal direction of the incontinence pad 1. The side leakage barriers 9 are made from separate material strips folded in two, the fold edges 7 constituting the ridges of the side leakage barriers 9. The respective legs of the material strips folded in two are fixed to the covering layer 2 and constitute the fixed edges 19 of the side leakage barriers.

In the front portion 21 and rear portion 22 of the incontinence pad 1, the inner side leakage barriers 9 are folded down and connected to the covering layer 2 over their entire widths.

The inner side leakage barriers 9 comprise elastic elements 24 connected to the inner side leakage barriers 9 in a pretensioned state. The elastic elements 24 are preferably arranged adjacent to the free, unfixed edges of the inner side leakage barriers 9. When the pretensioned elastic elements 24 are released, they contract together with the free edges of the inner side leakage barriers 9, the inner side leakage barriers 9 being brought into an erect configuration away from the liquid-permeable covering layer 2 in the crotch portion 23 of the incontinence pad 1, where the side leakage barriers 9 are connected to the covering layer 2 only at their respective free edges.

The rear and/or front portion(s) of the incontinence pad 1 can also be provided with what is known as waist elastic 25 which consists of elastic means arranged along the front transverse edge 14 and/or the rear transverse edge 15 of the incontinence pad 1 so that the diaper encloses the waist of the wearer gently and flexibly. In the present illustrative embodiment, only the rear end portion 22 of the incontinence pad 1 is provided with waist elastic 25 in the form of a thin strip of an elastic foam material which is attached by glue between the backing layer 4 and the liquid-permeable surface layer 2. The waist elastic 25 is applied in a stretched state between the layers in order to bring about a gathering force which stretches the incontinence pad 1 around the waist of the wearer.

The incontinence pad 1 is characterized mainly in that it comprises a fastening arrangement which fastens the incontinence pad 1 around the waist of the wearer. The fastening arrangement consists of a first fastening device 26 and a second fastening device 27. The fastening devices 26, 27 interconnect the front end portion 21 of the incontinence pad 1 with the rear end portion 22 when the incontinence pad is fitted on the wearer, the incontinence pad 1 taking on a pant-like configuration. The fastening devices 26, 27 are arranged so that they come to lie on the left and, respectively, right side of the wearer in the waist region when the incontinence pad is fitted on the wearer.

The first fastening device 26 consists of a first male part 28 and a first female part 29, and the second fastening device 27 consists of a second male part 30 and a second female part 31, the female parts 29, 31 being designed to be interconnected with the respective male part 28, 30.

The male part 28 and female part 29 of the first fastening device 26 are connected to the longitudinal edge 13 of the incontinence pad 1, and the male part 30 and female part 31 of the second fastening device 27 are connected to the opposite longitudinal edge 12 of the incontinence pad 1.

The male parts 28, 30 are arranged diagonally in the end portions 21, 22 of the incontinence pad 1, one male part 28 being arranged in the front end portion 21, and the other male part 30 being arranged in the rear end portion 22.

The female parts 29, 31 are also arranged diagonally in the end portions 21, 22 of the incontinence pad 1, one female part 29 being arranged in the rear end portion 22 and the other female part 31 being arranged in the front end portion 21.

The male parts 28, 30 and the female parts 29, 31 are connected directly to the longitudinal edges 12, 13 but can, in alternative embodiments, be connected via intermediate means arranged between the longitudinal edges 12, 13 and the respective male parts 28, 30 and female parts 29, 31. In this connection, the intermediate means can be inelastic or elastic and consist of, for example, one or more soft non-woven layers, or of one or more plastic film layers. Laminates consisting of non-woven layers and plastic film layers are also conceivable.

In an alternative embodiment, the male parts 28, 30 and female parts 29, 31 of the fastening devices 26, 27 can comprise the liquid-permeable covering layer 2 of the incontinence pad 1 or the backing layer 4, or both layers. In an embodiment where the liquid-permeable covering layer 2 and/or the backing layer 4 of the incontinence pad 1 extend(s) to constitute part of the male parts 28, 30 and/or female parts 29, 31 of the fastening devices 26, 27, advantages in terms of process engineering are obtained in that steps in which a number of separate parts have to be joined to one another are avoided. In the case of male parts 28, 30, the liquid-permeable covering layer 2 and/or the backing layer 4 will then extend to form the shape of the male parts 28, 30. If both the liquid-permeable covering layer 2 and the backing layer 4 extend, they are attached to one another in a corresponding way to that described with regard to connection around the periphery of the absorbent body 6. The female parts 29, 31 are formed in a corresponding way, with the exception that, in a corresponding region, the liquid-permeable covering layer 2 and the backing layer 4 will not be attached to one another but will form a first projecting tongue 32 and a second projecting tongue 33, which tongues are described below.

The female parts 29, 31 of the fastening devices 26, 27 can, for example, comprise one or more soft non-woven layers, one or more plastic film layers, or a laminate consisting of a combination of non-woven layers and plastic film layers or the like. It is also advantageous if those surfaces of the female parts 29, 31 which are oriented towards the wearer consist of a textile-like material made of, for example, non-woven, because such materials are usually more comfortable to wear against the skin than plastic film layers.

From an aesthetic point of view, it is also advantageous for those surfaces of the female parts 29, 31 which are oriented away from the wearer to consist of a textile-like material made of, for example, non-woven, especially when the incontinence pad 1 is of pant type.

The male parts 28, 30 of the fastening devices 26, 27 can, for example, comprise one or more soft non-woven layers, one or more plastic film layers, or a laminate consisting of a combination of non-woven layers and plastic film layers or the like. It is not necessary, but may be an advantage for interconnection of a fastening device 26, 27 according to the invention, if the male part 28, 30 has a certain rigidity so that it has a certain resistance to creasing.

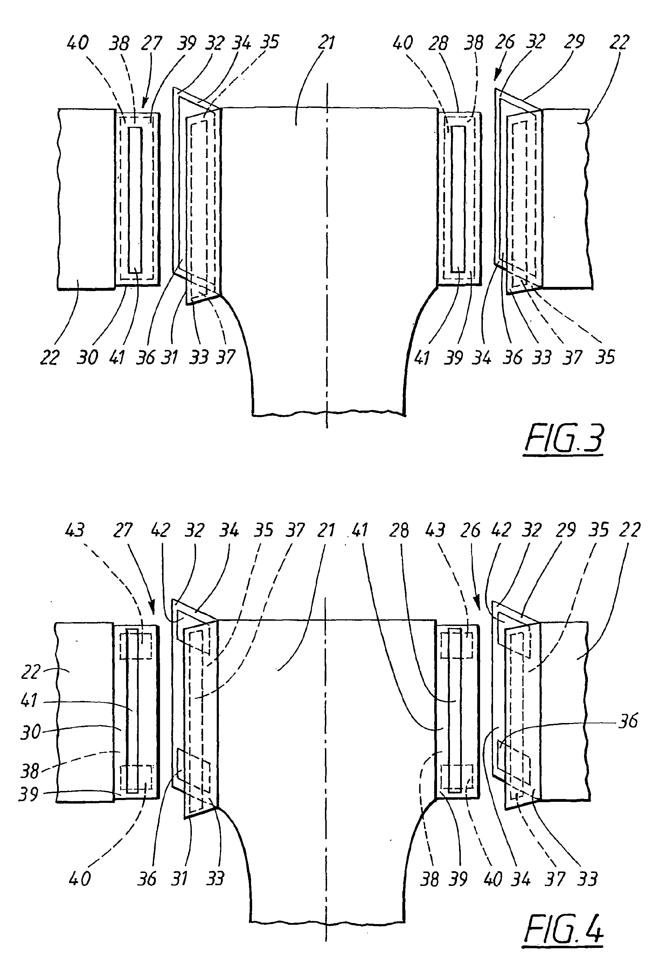

Figure 3 shows an enlarged illustration of a first embodiment of the two fastening devices 26, 27 of the incontinence pad 1 before interconnection around the waist of a wearer. The figure shows the front end portion 21 in its entirety, while only the parts of the rear end portion 22 which are connected to the respective female part 29 and male part 30 are shown.

The female part 29 of the first fastening device 26 is connected to the rear end portion 22 of the incontinence pad 1, and its male part 28 is connected to the front end portion 21.

The female part 31 of the second fastening device 27 is connected to the front end portion 21 of the incontinence pad 1, and its male part 30 is connected to the rear end portion 22.

Each female part 29, 31 has a first projecting tongue 32 and a second projecting tongue 33 connected to the liquid-permeable covering layer 2 and backing layer 4 of the incontinence pad 1. In this regard, a surface 34 on each first projecting tongue 32 is oriented towards a surface 35 on the respective second projecting tongue 33. A first fixing element 36 is arranged on the surface 34, and a second fixing element 37 is arranged on the surface 35.

Each male part 28, 30 has a first surface 38 and a second surface 39. A third fixing element 40, intended to interact with the first fixing element 36, is arranged on the first surface 38 of each male part 28, 30. A fourth fixing element 41, intended to interact with the second fixing element 37, is arranged on the second surface 39 of each male part 28, 30.

The fixing elements 36, 37, 40, 41 are of the hook and loop type, interacting fixing elements 36, 40 and 37, 41 consisting in pairs of a fixing element with hooks and a fixing element with loops.

In alternative embodiments, the fixing elements 36, 37, 40, 41 can consist of adhesive elements, interacting fixing elements 36, 40 and 37, 41 consisting in pairs of a fixing element with an adhesive surface and a fixing element with a receiving surface for the adhesive. In such adhesive embodiments, it is suitable for the adhesive and the receiving surface to be designed so that the connection between the surfaces can be opened and reclosed. Openable adhesive elements are often brought about by adhesive surfaces of what is known as the pressure-sensitive type which are applied to receiving surfaces treated with release agent, the adhesive connection then being openable. It is usual for release-agent treatment to be brought about by silicone treatment of the surface concerned.

For interacting adhesive fixing elements, it is suitable for the receiving surface to be slightly larger than the adhesive surface so that the whole adhesive surface is covered by the receiving surface.

Combination arrangements are also conceivable, where one pair of the interacting fixing elements 36, 40 or 37, 41 is of hook and loop type and the other pair is of adhesive type.

When the incontinence pad 1 is to be fitted on a wearer, the trunk of the wearer is first enclosed by the incontinence pad 1 with the rear end portion 22 at the rear on the wearer, the front end portion 21 at the front and the narrower crotch portion 23 between the thighs of the wearer. When this initial fitting has been completed, the joining-together of the male parts 28, 30 and female parts 29, 31 of the two fastening devices 26, 27 is started, all the component parts of one of the fastening devices 26, 27 being joined together before the joining-together of the other fastening device 26, 27 is started.

The first of the fastening devices 26, 27 is joined together by first interconnecting the first fixing element 36 with the third fixing element 40, and then interconnecting the second fixing element 37 with the fourth fixing element 41.

As this type of fastening device 26, 27 is often used for incontinence pads 1 which are after fitting to imitate a pair of pants, the fastening devices 26, 27 have a great extent in the longitudinal direction of the incontinence pad 1. A great extent of the fastening devices 26, 27 in the longitudinal direction of the incontinence pad 1, that is to say wide fastening devices 26, 27, involves difficulties in interconnecting the various fixing elements 36, 37, 40, 41, without creases and other irregularities being formed. Creases and other types of defect in an interconnected fastening device 26, 27 involve above all a risk of the incontinence pad 1 causing chafing against the skin of the wearer, but also of the incontinence pad 1 being felt to be less aesthetically attractive and thus less pant-like. The problem of creasing is especially great when component materials in the female parts 29, 31 and the male parts 28, 30 consist of soft flexible materials. In order to minimize the risk of obtaining a creased and uncomfortable interconnection of the wide fastening devices 26, 27, it is therefore suitable to use both hands when two fixing elements 36, 37, 40, 41 are interconnected, it then being possible for stretching-out/smoothing-out in the width direction to be effected when interconnection takes place.

The most usual method applied when the fastening devices 26, 27 of an incontinence pad 1 are to be interconnected on an incontinent wearer confined to bed is first to interconnect the fastening device 26, 27 which is oriented towards that side of the bed on which the carer is located. The patient is then rolled through a quarter of a turn towards the carer, after which the remaining fastening device 26, 27 is interconnected without the carer moving to the opposite side of the bed.

Owing to the fact that the fastening devices 26, 27 of the incontinence pad are arranged diagonally on the incontinence pad 1, the interconnection sequence described above for the two fastening devices 26, 27 means that the operations performed during interconnection of the first fastening device 2b, 27 are repeated in exactly the same way with the same hand for the second fastening device 26, 27. The carer has either the two male parts 28, 30 or the two female parts 29, 31 of the fastening devices 26, 27 oriented towards him/her during interconnection.

Depending on whether the carer is right-handed or left-handed, it may be more suitable to carry out the interconnection of the fastening devices 26, 27 of the incontinence pad 1 from the right or the left side of the patient and the bed.

The possibility of interconnecting the two wide and relatively complicated fastening devices 26, 27 in exactly the same way with exactly the same operation sequence has been found to improve the interconnection result considerably compared with if corresponding complex fastening devices 26, 27 were arranged conventionally on the incontinence pad 1. Conventionally arranged fastening devices 26, 27 mean that both the female parts 29, 31 are arranged in either the front or rear end portion 21, 22 of the incontinence pad 1 and that the male parts 28, 30 are arranged in the opposite end portion 21, 22. When such conventionally arranged fastening devices 26, 27 are to be interconnected from the same side of the patient, this involves the interconnection of mirror-inverted fastening devices 26, 27, which often results in an inferior interconnection result.

Figure 4 shows an enlarged illustration of a second embodiment of the two fastening devices 26, 27 of the incontinence pad 1 before interconnection around the waist of a wearer. Reference numbers indicated in Figure 4 correspond to equivalent reference numbers for the first embodiment in Figure 3.

On the first projecting tongue 32 of each female part 29, 31, a fifth fixing element 42 is arranged on the same surface 34 as the first fixing element 36. In this connection, the first and the fifth fixing elements 36, 42 are arranged at a distance from one another in the longitudinal direction of the incontinence pad 1.

A sixth fixing element 43 is arranged on the first surface 38 of each male part 28, 30. In this connection, the two fixing elements 40, 43 which are arranged on the first surface 38 of each male part 28, 30 are arranged at a distance from one another in the longitudinal direction of the incontinence pad 1.

In this connection, the distance in the longitudinal direction between the first and fifth fixing elements 36, 42 is essentially the same as the distance between the third and sixth fixing elements 40, 43.

The advantage of an incontinence pad 1 comprising fastening devices 26, 27 according to this second embodiment is that it can feel slightly softer and more flexible to the wearer than the fastening devices 26, 27 according to the first embodiment. However, the price to pay for this softness and flexibility is that it may, especially before one has become accustomed to it, be a shade more difficult to interconnect the fastening devices 26, 27 because a further two fixing elements 42, 43 have to be interconnected in each case.

Fitting of the incontinence pad 1 according to this embodiment is carried out in the same way as the fitting of an incontinence pad 1 according to the first embodiment, that is to say enclosing the trunk of the wearer with the incontinence pad 1, interconnection of one of the fastening devices 26, 27, followed by interconnection of the other fastening device 26, 27.

The sequence for interconnection of the fastening devices 26, 27 of the incontinence pad 1 nevertheless differs slightly from the sequence in the first embodiment.

The sequence for interconnection of each fastening device 26, 27 according to the second embodiment is first to interconnect the first and third fixing elements 36, 40, and then to interconnect the fifth and sixth fixing elements 42, 43. Finally, the second fixing element 37 is interconnected with the fourth fixing element 41. Before the last operation in the interconnection sequence, that is to say interconnection of the fifth and sixth fixing elements 42, 43, for each fastening device 26, 27, it is important to ensure that the first projecting tongue 32 of the female parts 29, 31 and the male parts 28, 30 are properly stretched and smooth. If this is not ensured, there is a great risk of creases being formed during the final interconnection between the second and fourth fixing elements 37, 41, the fastening device 26, 27 then being both uncomfortable and not aesthetically attractive.

Fastening devices 26, 27 according to the invention are slightly more complex to fit than conventional fastening devices but at the same time represent considerably improved fastening devices 26, 27. The improvements are above all in wearer comfort and appearance, but also in the strength of the interconnected units.

Incontinence pads 1 of what is known as the openable pant type are a type of incontinence pad 1 which, after fitting on a wearer, is to be perceived by both the wearer and other people as what are known as incontinence pants. Incontinence pants already have a continuous waist region when they are delivered and are put on the wearer in precisely the same way as a pair of ordinary pants, that is to say slipped over the feet and pulled up along the legs. Such incontinence pants therefore require no joining-together of a front and rear waist region at the time of fitting.

It is therefore important for an incontinence pad 1 of the openable pant type that the joining-together of the front and rear waist regions produces a join which is both comfortable for the wearer and more or less invisible. Such joins usually have to be considerably wider than joins on conventional what are known as all-in-one diapers. In order to be invisible, the joins also have to be covered by a material layer of the same texture as the material layer which constitutes the outside of the incontinence pad 1.

The invention includes all conceivable combinations of the illustrative embodiments described.

Moreover, the invention is not limited to the illustrative embodiments above, but is of course applicable for other embodiments within the scope of the patent claims below.

## Claims

1. Absorbent article (1) such as a diaper, an incontinence pad or a sanitary towel, comprising an absorbent body (6), a covering (16) comprising a liquid-permeable covering layer (2) and a backing layer (4), and a fastening arrangement for fastening the article on a wearer, the fastening arrangement comprises a first and a second fastening device (26, 27), each fastening device (26, 27) comprising a female part (29, 31) and a male part (28, 30), the female part (29, 31) having a first projecting tongue (32) connected to the covering (16), and a second projecting tongue (33) connected to the covering (16), a surface on the first projecting tongue (32) being oriented towards a surface on the second projecting tongue (33), and a first fixing element (36) being arranged on that surface of the first projecting tongue (32) which is oriented towards the second projecting tongue (33), and a second fixing element (37) being arranged on that surface of the second projecting tongue (33) which is oriented towards the first projecting tongue (32), and the male part (28, 30) being connected to the covering (16), the male part (28, 30) having a first surface (38) comprising a third fixing element (40), and a second surface (39) comprising a fourth fixing element (41), the male part (28, 30) and the female part (29, 31) being interconnectable, the first fixing element (36) interacting with the third fixing element (40) and the second fixing element (37) interacting with the fourth fixing element (41), **characterized in that** the respective female parts (29, 31) of the fastening devices (26, 27) are arranged in diagonally opposite corners of the article (1), and **in that** the respective male parts (28, 30) of the fastening devices (26, 27) are arranged in the other two diagonally opposite corners of the article (1) when the article is in a folded-out state.

2. Absorbent article (1) according to Claim 1, **characterized in that** a fifth fixing element (42) is arranged on that surface of the first projecting tongue (32) of the female part (29, 31) which is oriented towards the second projecting tongue (33) and a sixth fixing element (43) is arranged on the first surface of the male part (28, 30), the fifth fixing element (42) being interconnectable with the sixth fixing element (43).

3. Absorbent article (1) according to Claim 1 or Claim 2, **characterized in that** the fastening devices (26, 27) comprise mechanical fixing elements.

4. Absorbent article (1) according to Claim 3, **characterized in that** the mechanical fixing elements consist of hook and loop elements.

5. Absorbent article (1) according to Claim 1 or Claim 2, **characterized in that** the fastening devices (26, 27) comprise adhesive fixing elements.

6. Absorbent article (1) according to any one of the preceding claims; **characterized in that** the male parts (28, 30) of the fastening devices (26, 27) comprise the liquid-permeable covering layer (2) of the incontinence pad (1) or the backing layer (4), or both layers.

7. Absorbent article (1) according to any one of the preceding claims, **characterized in that** the female parts (29, 31) of the fastening devices (26, 27) comprise the liquid-permeable covering layer (2) of the incontinence pad (1) or the backing layer (4), or both layers.

8. Absorbent article (1) according to any one of the preceding claims, **characterized in that** the article is an openable pant product.

9. Method for fitting an absorbent article (1) comprising a fastening arrangement according to any one of the preceding claims on a wearer, **characterized in that** interconnection of the two fastening devices (26, 27) of the fastening arrangement is carried out from the same position in relation to the article (1) and the wearer of the article, the sequence of operations for interconnection of the fastening device (26, 27) selected first and the fastening device (26, 27) selected subsequently being identical, the same hand being used for the same operations in the sequence for both the fastening device (26, 27) selected first and the fastening device (26, 27) selected subsequently.

10. Method according to Claim 9 for fitting an absorbent article (1) comprising a fastening arrangement according to any one of Claims 1, 3, 4, 5 or 6 on a wearer, **characterized in that** interconnection of the fastening device (26, 27) selected first is carried out by first interconnecting the first fixing element (36) with the third fixing element (40) and then interconnecting the second fixing element (37) with the fourth fixing element (41), after which interconnection of the fixing elements (36, 40, 37, 41) for the fastening device (26, 27) selected subsequently is carried out in the same order as the order for the fixing elements (36, 40, 37, 41) of the fastening device (26, 27) selected first.

11. Method according to Claim 9 for fitting an absorbent article (1) comprising a fastening arrangement according to any one of Claims 2, 3, 4, 5 or 6 on a wearer, **characterized in that** interconnection of the fastening device (26, 27) selected first is carried out by first interconnecting the first fixing element (36) with the third fixing element (40) and then interconnecting the fifth fixing element (42) with the sixth fixing element (43), after which the second fixing element (37) is interconnected with the fourth fixing element (41), after which interconnection of the fixing elements (36, 40, 42, 43, 37, 41) for the fastening device (26, 27) selected subsequently is carried out in the same order as the order for the fixing elements (36, 40, 42, 43, 37, 41) of the fastening device (26, 27) selected first.

12. Method according to Claim 9 for fitting an absorbent article (1) comprising a fastening arrangement according to any one of Claims 2, 3, 4, 5 or 6 on a wearer, **characterized in that** interconnection of the fastening device (26, 27) selected first is carried out by first interconnecting the fifth fixing element (42) with the sixth fixing element (43) and then interconnecting the first fixing element (36) with the third fixing element (40), after which the second fixing element (37) is interconnected with the fourth fixing element (41), after which interconnection of the fixing elements (42, 43, 36, 40, 37, 41) for the fastening device (26, 27) selected subsequently is carried out in the same order as the order for the fixing elements (42, 43, 36, 40, 37, 41) of the first fastening device (26, 27).

## Patentansprüche

1. Absorptionsartikel (1), wie z.B. eine Windel, ein Inkontinenzpad oder eine Binde, umfassend einen Absorptionskörper (6), eine Abdeckung (16) mit einer Flüssigkeitsdurchlässigen Abdeckschicht (2) und einer Rückenschicht (4) und einer Befestigungsanordnung zum Befestigen des Artikels an einem Träger, wobei die Befestigungsanordnung eine erste und eine zweite Befestigungsvorrichtung (26, 27) umfasst, wobei jede Befestigungsvorrichtung (26, 27) einen weiblichen Teil (29, 31) und einen männlichen Teil (28, 30) aufweist, wobei der weibliche Teil (29, 31) eine erste vorstehende Zunge (32), die mit der Abdeckung (16) verbunden ist, und eine zweite vorstehende Zunge (33), die mit der Abdeckung (16) verbunden ist, aufweist, wobei eine Oberfläche auf der ersten vorstehenden Zunge (32) in Richtung einer Oberfläche auf der zweiten vorstehenden Zunge (33) ausgerichtet ist, und ein erstes Befestigungselement (36), das auf der Oberfläche der ersten vorstehenden Zunge (32) angeordnet ist, die in Richtung der zweiten vorstehenden Zunge (33) ausgerichtet ist, und ein zweites Befestigungselement (37), das auf der Oberfläche der zweiten vorstehenden Zunge (33) angeordnet ist, die in Richtung der ersten vorstehenden Zunge (32) ausgerichtet ist, und der männliche Teil (28, 30) mit der Abdeckung (16) verbunden ist, wobei der männliche Teil (28, 30) eine erste Oberfläche (38) mit einem dritten Befestigungselement (40) und eine zweite Oberfläche (39) mit einem vierten Befestigungselement (41) aufweist, wobei der männliche Teil (28, 30) und der weibliche Teil (29, 31) miteinander verbunden werden können, wobei das erste Befestigungselement (36) mit dem dritten Befestigungselement (40) zusammenwirkt und das zweite Befestigungselement (37) mit dem vierten Befestigungselement (41) zusammenwirkt, **dadurch gekennzeichnet, dass** die jeweiligen weiblichen Teile (37) mit dem vierten Befestigungselement (41) zusammenwirken, **dadurch gekennzeichnet, dass** die jeweiligen weiblichen Teile (29, 31) der Befestigungsvorrichtungen (26, 27) in diagonal gegenüberliegenden Ecken des Artikels (1) angeordnet sind und dass die entsprechenden männlichen Teile (28, 30) der Befestigungsvorrichtungen (26, 27) in den anderen beiden diagonal gegenüberliegenden Ecken des Artikels (1) angeordnet sind, wenn der Artikel in einem entfalteten Zustand ist.

2. Absorptionsartikel (1) nach Anspruch 1, **dadurch gekennzeichnet, dass** ein fünftes Befestigungselement (42) auf der Oberfläche der ersten vorstehenden Zunge (32) des weiblichen Teils (29, 31) angeordnet ist, die in Richtung der zweiten vorstehenden Zunge (33) ausgerichtet ist, und ein sechstes Befestigungselement (43) auf der ersten Oberfläche des männlichen Teils (28, 30) angeordnet ist, wobei das fünfte Befestigungselement (42) mit dem sechsten Befestigungselement (43) verbunden werden kann.

3. Absorptionsartikel (1) nach Anspruch 1 oder Anspruch 2, **dadurch gekennzeichnet, dass** die Befestigungsvorrichtungen (26, 27) mechanische Befestigungselemente umfassen.

4. Absorptionsartikel (1) nach Anspruch 3, **dadurch gekennzeichnet, dass** die mechanischen Befestigungselemente aus Haken- und Ösenelementen bestehen.

5. Absorptionsartikel (1) nach Anspruch 1 oder Anspruch 2, **dadurch gekennzeichnet, dass** die Befestigungsvorrichtungen (26, 27) klebende Befestigungselemente umfassen.

6. Absorptionsartikel (1) nach einem der vorhergehenden Ansprüche, **dadurch gekennzeichnet, dass** die männlichen Teile (28, 30) der Befestigungsvorrichtungen (26, 27) die flüssigkeitsdurchlässige Abdeckschicht (2) des Inkontinenzpads (1) oder die Rückenschicht (4) oder beide Schichten umfassen.

7. Absorptionsartikel (1) nach einem der vorhergehenden Ansprüche, **dadurch gekennzeichnet, dass** die weiblichen Teile (29, 31) der Befestigungsvorrichtungen (26, 27) die flüssigkeitsdurchlässige Abdeckschicht (2) des Inkontinenzpads (1) oder die Rückenschicht (4) oder beide Schichten umfassen.

8. Absorptionsartikel (1) nach einem der vorhergehenden Ansprüche, **dadurch gekennzeichnet, dass** der Artikel ein öffnungsfähiges Hosenprodukt ist.

9. Verfahren zum Anlegen eines Absorptionsartikels (1) mit einer Befestigungsanordnung nach einem der vorhergehenden Ansprüche an einem Träger, **dadurch gekennzeichnet, dass** eine Verbindung der zwei Befestigungsvorrichtungen (26, 27) der Befestigungsanordnung aus derselben Position in Bezug auf den Artikel (1) und den Träger des Artikels durchgeführt wird, wobei die Verfahrensfolgen zum Verbinden der Befestigungsvorrichtung (26, 27), die zuerst ausgewählt wird, und der Befestigungsvorrichtung (26, 27), die danach ausgewählt wird, identisch sind, wobei dieselbe Hand für dasselbe Verfahren in der Folge sowohl für die Befestigungsvorrichtung (26, 27), die zuerst ausgewählt wird, als auch für die Befestigungsvorrichtung (26, 27), die danach ausgewählt wird, verwendet wird.

10. Verfahren nach Anspruch 9, zum Befestigen eines Absorptionsartikels (1) mit einer Befestigungsanordnung nach einem der Ansprüche 1, 3, 4, 5 oder 6 an einem Träger, **dadurch gekennzeichnet, dass** die Verbindung der Befestigungsvorrichtung (26, 27), die zuerst ausgewählt wird, zuerst durch ein Verbinden des ersten Befestigungselements (36) mit dem dritten Befestigungselement (40) und dann ein Verbinden des zweiten Befestigungselements (37) mit dem vierten Befestigungselement (41) ausgeführt wird, wonach eine Verbindung der Befestigungselemente (36, 40, 37, 41) für die Befestigungsvorrichtung (26, 27), die danach ausgewählt wird, in derselben Reihenfolge wie die Reihenfolge der Befestigungselemente (36, 40, 37, 41) der zuerst ausgewählten Befestigungsvorrichtung (26, 27) durchgeführt wird.

11. Verfahren nach Anspruch 9 zum Anlegen eines Absorptionsartikels (1) mit einer Befestigungsanordnung nach einem der Ansprüche 2, 3, 4, 5 oder 6 an einem Träger, **dadurch gekennzeichnet, dass** eine Verbindung der Befestigungsvorrichtung (26, 27), die zuerst ausgewählt wird, zuerst durch Verbinden des ersten Befestigungselements (36) mit dem dritten Befestigungselement (40) und dann Verbinden des fünften Befestigungselements (42) mit dem sechsten Befestigungselement (43) durchgeführt wird, wonach das zweite Befestigungselement (37) mit dem vierten Befestigungselement (41) verbunden wird, wonach eine Verbindung der Befestigungselemente (36, 40, 42, 43, 37, 41) für die Befestigungsvorrichtung (26, 27), die danach ausgewählt wird, in derselben Reihenfolge wie der Reihenfolge der Befestigungselemente (36, 40, 42, 43, 37, 41) der zuerst ausgewählten Befestigungsvorrichtung (26, 27) durchgeführt wird.

12. Verfahren nach Anspruch 9 zum Befestigen eines Absorptionsartikels (1) mit einer Befestigungsanordnung nach einem der Ansprüche 2, 3, 4, 5 oder 6 an einem Träger, **dadurch gekennzeichnet, dass** eine Verbindung der Befestigungsvorrichtung (26, 27), die zuerst ausgewählt wird, zuerst durch Verbinden des fünften Befestigungselements (42) mit dem sechsten Befestigungselement (43) und dann Verbinden des ersten Befestigungselements (36) mit dem dritten Befestigungselement (40) durchgeführt wird, wonach das zweite Befestigungselement (37) mit dem vierten Befestigungselement (41) verbunden wird, wonach eine Verbindung der Befestigungselemente (42, 43, 36, 40, 37, 41) für die danach ausgewählte Befestigungsvorrichtung (26, 27) in derselben Reihenfolge wie der Reihenfolge der Befestigungselemente (42, 43, 36, 40, 37, 41) der ersten Befestigungsvorrichtung (26, 27) durchgeführt wird.

## Revendications

1. Article absorbant (1) tel qu'une couche, une couche d'incontinence ou une serviette hygiénique, comportant un corps absorbant (6), un revêtement (16) comprenant une couche de revêtement perméable aux liquides (2) et une couche de support (4) et un système de serrage pour serrer l'article sur la personne qui le porte, le système de serrage comprend un premier et un second dispositif de serrage (26, 27), chaque dispositif de serrage (26, 27) comportant une partie femelle (29, 31) et une partie mâle (28, 30), la partie femelle (29, 31) présentant une première languette en saillie (32) reliée au revêtement (16) et une seconde languette en saillie (33) reliée au revêtement (16), une surface située sur la première languette en saillie (32) étant orientée en direction d'une surface située sur la seconde languette en saillie (33) et un premier élément de fixation (36) étant agencé sur cette surface de la première languette en saillie (32) qui est orientée en direction de la seconde languette en saillie (33) et un second élément de fixation (37) étant agencé sur cette surface de la seconde languette en saillie (33) qui est orientée en direction de la première languette en saillie (32) et la partie mâle (28, 30) étant reliée au revêtement (16), la partie mâle (28, 30) présentant une première surface (38) comportant un troisième élément de fixation (40) et une seconde surface (39) comportant un quatrième élément de fixation (41), la partie mâle (28, 30) et la partie femelle (29, 31) pouvant être reliées, le premier élément de fixation (36) interagissant avec le troisième élément de fixation (40) et le second élément de fixation (37) interagissant avec le quatrième élément de fixation (41), **caractérisé en ce que** les parties femelles respectives (29, 31) des dispositifs de serrage (26, 27) sont disposées dans des coins de l'article (1), opposés dans le sens diagonal, et **en ce que** les parties mâles respectives (28, 30) des dispositifs de serrage (26, 27) sont disposées dans les deux autres coins, opposés dans le sens diagonal, de l'article (1) lorsque l'article est déplié.

2. Article absorbant (1) selon la revendication 1, **caractérisé en ce qu'**un cinquième élément de fixation (42) est agencé sur cette surface de la première languette en saillie (32) de la partie femelle (29, 31) qui est orientée en direction de la seconde languette en saillie (33) et un sixième élément de fixation (43) est agencé sur la première surface de la partie mâle (28, 30), le cinquième élément de fixation (42) pouvant être relié au sixième élément de fixation (43).

3. Article absorbant (1) selon la revendication 1 ou la revendication 2, **caractérisé en ce que** les dispositifs de serrage (26, 27) comprennent des éléments de fixation mécaniques.

4. Article absorbant (1) selon la revendication 3, **caractérisé en ce que** les éléments de fixation mécanique sont composés d'éléments à velcro.

5. Article absorbant (1) selon la revendication 1 ou la revendication 2, **caractérisé en ce que** les dispositifs de serrage (26, 27) comprennent des éléments de fixation adhésifs.

6. Article absorbant (1) selon l'une quelconque des revendications précédentes, **caractérisé en ce que** les parties mâles (28, 30) des dispositifs de serrage (26, 27) comprennent la couche de revêtement perméable aux liquides (2) de la couche d'incontinence (1) ou la couche de support (4) ou les deux couches.

7. Article absorbant (1) selon l'une quelconque des revendications précédentes, **caractérisé en ce que** les parties femelles (29, 31) des dispositifs de serrage (26, 27) comprennent la couche de revêtement perméable aux liquides (2) de la couche d'incontinence (1) ou la couche de support (4) ou les deux couches.

8. Article absorbant (1) selon l'une quelconque des revendications précédentes, **caractérisé en ce que** l'article est un produit à mode de slip pouvant être ouvert.

9. Procédé de fixation d'un article absorbant (1), comportant un système de serrage selon l'une quelconque des revendications précédentes, sur une personne, **caractérisé en ce que** la liaison des deux dispositifs de serrage (26, 27) du système de serrage est réalisée à partir de la même position en fonction de l'article (1) et de la personne qui porte l'article, la séquence des opérations de liaison du dispositif de serrage (26, 27) sélectionné en premier et du second dispositif de serrage (26, 27) sélectionné après étant identiques, la même main étant utilisée pour les mêmes opérations dans la séquence à la fois pour le dispositif de serrage (26, 27) sélectionné en premier et le dispositif de serrage (26, 27) sélectionné après.

10. Procédé selon la revendication 9 pour fixer un article absorbant (1), comportant un système de serrage selon l'une quelconque des revendications 1, 3, 4, 5 ou 6 sur une personne, **caractérisé en ce que** la liaison du dispositif de serrage (26, 27) sélectionné en premier est réalisée par le fait de relier tout d'abord le premier élément de fixation (36) au troisième élément de fixation (40) et de relier ensuite le second élément de fixation (37) au quatrième élément de fixation (41) après quoi on effectue la liaison des éléments de fixation (36, 40, 37, 41), pour le dispositif de serrage (26, 27) sélectionné après, dans le même ordre que celui employé pour les éléments de fixation (36, 40, 37, 41) du dispositif de serrage (26, 27) sélectionné en premier.

11. Procédé selon la revendication 9 pour fixer un article absorbant (1), comportant un système de serrage selon l'une quelconque des revendications 2, 3, 4, 5 ou 6 sur une personne, **caractérisé en ce que** la liaison du dispositif de serrage (26, 27) sélectionné en premier est réalisée par le fait de relier tout d'abord le premier élément de fixation (36) au troisième élément de fixation (40) et de relier ensuite le cinquième élément de fixation (42) au sixième élément de fixation (43), après quoi on effectue la liaison du second élément de fixation (37) au quatrième élément de fixation (41), après quoi on réalise la liaison des éléments de fixation (36, 40, 42, 43, 37, 41) pour le dispositif de serrage (26, 27) sélectionné après, dans le même ordre que celui employé pour les éléments de fixation (36, 40, 42, 43, 37, 41) du dispositif de serrage (26, 27) sélectionné en premier.

12. Procédé selon la revendication 9 pour fixer un article absorbant (1), comportant un système de serrage selon l'une quelconque des revendications 2, 3, 4, 5 ou 6 sur une personne, **caractérisé en ce que** la liaison du dispositif de serrage (26, 27) sélectionné en premier est réalisée par le fait de relier tout d'abord le cinquième élément de fixation (42) au sixième élément de fixation (43) et de relier ensuite le premier élément de fixation (36) au troisième élément de fixation (40), après quoi on réalise la liaison du second élément de fixation (37) au quatrième élément de fixation (41), après quoi on effectue la liaison des éléments de fixation (42, 43, 36, 40, 37, 41) pour le dispositif de serrage (26, 27) sélectionné après, dans le même ordre que celui employé pour les éléments de fixation (42, 43, 36, 37, 41) du premier dispositif de serrage (26, 27).
